# EUROPEAN PATENT APPLICATION

(11) **EP 4 310 501 A1**
(43) Date of publication of application: **24.01.2024**
(21) Application number: 22771495.3
(22) Date of filing: 17.03.2022
(51) Int. Cl.: G01N 33/68, C07K 14/705, C07K 16/28, C12N 15/09, C12Q 1/68, C12Q 1/6886, G01N 33/53

(54) **COMPANION MARKER FOR ASSESSMENT METHOD OF LYMPH NODE METASTASIS ABILITY OF ENDOMETRIAL CANCER**

(30) Priority: 19.03.2021 JP 2021046188
(71) Applicant: Juntendo Educational Foundation, Tokyo 113-8421 (JP); Kanagawa Prefectural Hospital Organization, Kanagawa 231-0005 (JP)
(72) Inventor: TERAO, Yasuhisa, Tokyo 113-8421 (JP); YOSHIDA, Emiko, Tokyo 113-8421 (JP); TAKEUCHI, Shiori, Tokyo 113-8421 (JP); HORIMOTO, Yoshiya, Tokyo 113-8421 (JP); ARAKAWA, Atsushi, Tokyo 113-8421 (JP); ITO, Masayoshi, Wako-shi, Saitama 351-0198 (JP); USUI, Kengo, Wako-shi, Saitama 351-0198 (JP); KOGO, Yasushi, Wako-shi, Saitama 351-0198 (JP); KATO, Hisamori, Yokohama-shi, Kanagawa 241-8515 (JP); OHTSU, Takashi, Yokohama-shi, Kanagawa 241-8515 (JP); UENO, Yuta, Yokohama-shi, Kanagawa 241-8515 (JP); KATO, Tomoyasu, Tokyo 104-0045 (JP)
(74) Representative: Plougmann Vingtoft a/s
(86) International application number: PCT/JP2022/012164
(87) International publication number: WO 2022/196750

(57) **Abstract**

Provided are a method for assessing recurrence risk of endometrial cancer and a method for using the same. A method for assessing recurrence risk of endometrial cancer, including the step of measuring an expression level of at least one selected from the group consisting of PGR and ERa in a biological sample derived from cancer tissue isolated from an endometrial cancer patient. A method for assessing lymph node metastasis or lymph node metastatic potential or prognosis of endometrial cancer, including the step of measuring, in a biological sample derived from a cancer tissue isolated from an endometrial cancer patient, an expression level of at least one selected from the group consisting of PGR and ERa and an expression level of at least one selected from the group consisting of SEMA3D and TACC2.

## Description

### Technical Field

### (RELATED APPLICATIONS AND INCORPORATION BY REFERENCE)

This application claims priority to Japanese Patent Application No. 2021-046188 filed on March 19, 2021, the entire contents of which are incorporated herein by reference.

Also, all literature cited herein is incorporated by reference in their entirety for all purposes. The citation of any literature is not to be construed as an admission that it is prior art with respect to the present invention.

The present invention relates to a companion marker for a method for assessing lymph node metastatic potential of endometrial cancer and use thereof.

### Background Art

In recent years, cancers in the gynaecological field have increased in various countries including Japan, and in particular, endometrial cancer is the most common cancer in the same field excluding cervical cancer. As a standard initial treatment for early endometrial cancer, surgical therapy is the first choice, but it is difficult to examine in advance the presence or absence of lymph node metastasis, which is a risk factor and also a criterion for determining advanced stage,in endometrial cancer. Therefore, lymph node dissection is performed as a standard surgical method in addition to simple total hysterectomy and adnexectomy, and determination of advanced stage and classification of recurrence risk are performed by postoperative pathological diagnosis. However, not only there is a problem of side effects due to lymph node dissection such as refractory lymphedema, but also there is a potential problem in the material itself for determination of definite diagnosis, such that the pathological diagnosis depends on morphological determination of an individual diagnostician, and there is a possibility that micrometastasis is overlooked in principle.

About 80% of endometrial cancers are cancers with low or moderate risk of recurrence and have a good prognosis, and about 10% of these cancers cause lymph node metastasis. On the other hand, the remaining about 20% has a high recurrence risk, and lymph node metastasis occurs with high frequency. Considering the problem of side effects of lymph node dissection, it is desirable to avoid unnecessary lymph node dissection, and for this purpose, a practical and accurate method of assessing lymph node metastasis without performing lymph node dissection, particularly such a method for cancer with low or moderate risk of recurrence is required.

As such a technique, the present inventors have heretofore found Semaphorin 3D (SEMA3D) and Transforming acidic coiled-coil-containing protein 2 (TACC2) as molecular markers for assessing the presence or absence or potential or prognosis of lymph node metastasis of endometrial cancer (Patent Literatures 1 and 2). The marker enables highly accurate assessment particularly for endometrial cancer patients with low or moderate risk of recurrence. Therefore, if the recurrence risk can be determined by measuring a specimen obtained by primary curettage before surgery or immediately measuring a cancer primary lesion extracted by surgery, and a patient with low or moderate risk of recurrence can be selected, assessment by the marker can be performed with higher accuracy. As a result, unnecessary lymph node dissection can be omitted and the patient can be relieved of the risk of refractory complications. In addition, it can be expected to realize effective personalized medical care having great merit for patients.

Progesterone receptor (PGR) and estrogen receptor α (ERα) are one type of hormone receptors, and are used as indices for determining whether a cancer is a hormonedependent cancer in which hormone therapy is effective in breast cancer and the like.

### Citation List

### Patent Literatures

Patent Literature 1: WO 2015/105190
Patent Literature 2: WO 2017/007031

### Summary of Invention

### Technical Problem

The present invention relates to identification of a marker molecule for determining recurrence risk of endometrial cancer, and provision of a method for determining recurrence risk of endometrial cancer by measuring the marker, and a method for molecularly discriminating between lymph node metastatic endometrial cancer and non-metastatic endometrial cancer by measuring the marker and a marker for discriminating between lymph node metastatic endometrial cancer and non-metastatic endometrial cancer.

### Solution to Problem

The present inventors have found that, as a result of extracting RNA from cancer tissues of endometrial cancer patients and comprehensively analyzing the expression state near the transcription start site as sequence information using a CAGE (cap analysis gene expression) analysis method, the expression level of DNA containing the transcription start site of PGR is significantly different between a group with low or moderate recurrence risk and no lymph node metastasis and the other groups, and as a result of examining using a TCGA (The Cancer Genome Atlas) database, the expression levels of PGR and ERa are significantly different between the group with low or moderate recurrence risk and no lymph node metastasis and the other groups. In addition, the present inventors have found that the expression levels of PGR and ERa are significantly different between the group with low or moderate recurrence risk and a group with high recurrence risk, and that the recurrence risk of endometrial cancer can be determined using these as indices. Furthermore, the present inventors have found that by selecting a specimen with low or moderate recurrence risk based on the expression levels of PGR and ERa and using the specimen as a subject, it is possible to accurately discriminate between lymph node metastatic endometrial cancer and non-metastatic endometrial cancer using the expression levels of SEMA3D and TACC2 as indices.

That is, the present invention provides the following [1] to [8] .
[1] A method for assessing recurrence risk of endometrial cancer, including the step of measuring an expression level of at least one selected from the group consisting of PGR and ERa in a biological sample derived from cancer tissue isolated from an endometrial cancer patient.
[2] A method for assessing tissue malignancy of endometrial cancer, including the step of measuring an expression level of at least one selected from the group consisting of PGR and ERa in a biological sample derived from a cancer tissue isolated from an endometrial cancer patient.
[3] A method for assessing lymph node metastasis or lymph node metastatic potential or prognosis of endometrial cancer, including the step of measuringan expression level of at least one selected from the group consisting of PGR and ERa and an expression level of at least one selected from the group consisting of SEMA3D and TACC2 in a biological sample derived from a cancer tissue isolated from an endometrial cancer patient.
[4] A test kit for assessing recurrence risk of endometrial cancer or tissue malignancy of endometrial cancer used in the method according to [1] or [2], including at least one antibody selected from the group consisting of an antibody that specifically binds to a PGR protein and an antibody that specifically binds to an ERa protein, or at least one oligonucleotide selected from the group consisting of an oligonucleotide that specifically recognizes a nucleic acid derived from a PGR gene and an oligonucleotide that specifically recognizes a nucleic acid derived from an ERa gene.
[5] A test kit for assessing lymph node metastasis or lymph node metastatic potential or prognosis of endometrial cancer used in the method according to [3], including at least one antibody selected from the group consisting of an antibody that specifically binds to a PGR protein and an antibody that specifically binds to an ERa protein, and at least one antibody selected from the group consisting of an antibody that specifically binds to a SEMA3D protein and an antibody that specifically binds to a TACC2 protein, or at least one oligonucleotide selected from the group consisting of an oligonucleotide that specifically recognizes a nucleic acid derived from a PGR gene and an oligonucleotide that specifically recognizes a nucleic acid derived from an ERa gene, and at least one oligonucleotide selected from the group consisting of an oligonucleotide that specifically recognizes a nucleic acid derived from a SEMA3D gene and an oligonucleotide that specifically recognizes a nucleic acid derived from a TACC2 gene.
[6] A device for assessing recurrence risk of endometrial cancer or tissue malignancy of endometrial cancer, used in the method according to [1] or [2].
[7] A device for assessing lymph node metastasis or lymph node metastatic potential or prognosis of endometrial cancer, used in the method according to [3].
[8] Use of at least one selected from the group consisting of PGR and ERα as a marker for assessing recurrence risk of endometrial cancer.

### Advantageous Effects of Invention

According to the present invention, an endometrial cancer specimen with low or moderate recurrence risk can be selected. Furthermore, by using the selected specimen as a subject, it is possible to highly accurately select an endometrial cancer specimen with no possibility of lymph node metastasis. As a result, a suggestion of a policy regarding a surgical method including avoidance of unnecessary lymph node dissection is obtained. If the lymph node dissection can be avoided, shortening of operation time, reduction of surgical invasion, postoperative lymphedema and the like can be prevented. That is, the marker of the present invention enables selection of a subject suitable for assessment of lymph node metastasis of endometrial cancer, and is useful as a companion marker contributing to improvement in accuracy of the assessment. The present invention can also be suitably used for a test performed by a medical worker in the vicinity of a patient from collection to analysis of a patient specimen (POCT: Point of Care Testing).

### Brief Description of Drawings

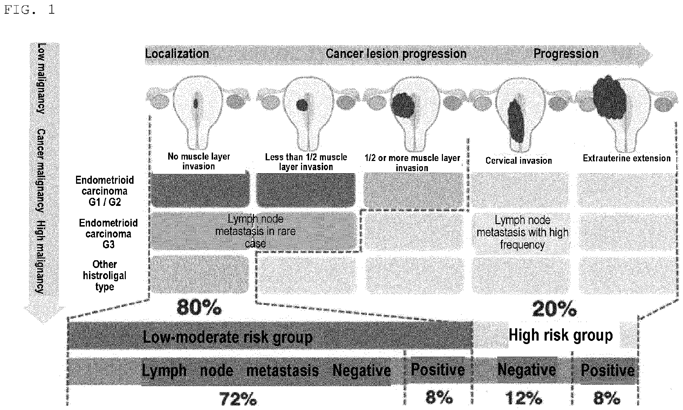

Fig. 1 indicates a relationship between tissue malignancy of endometrial cancer and progression of a cancer lesion.

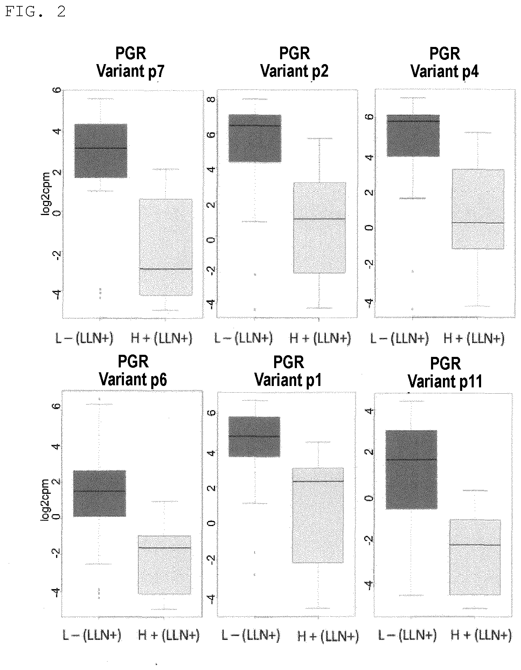

Fig. 2 indicates RNA expression levels of six variants of PGR in a lymph node metastasis negative group of a low to moderate risk group for recurrence (L-(LLN+)) and a lymph node metastasis positive group of a high risk group + a low to moderate risk group for recurrence (H+(LLN+)) of endometrial cancer.

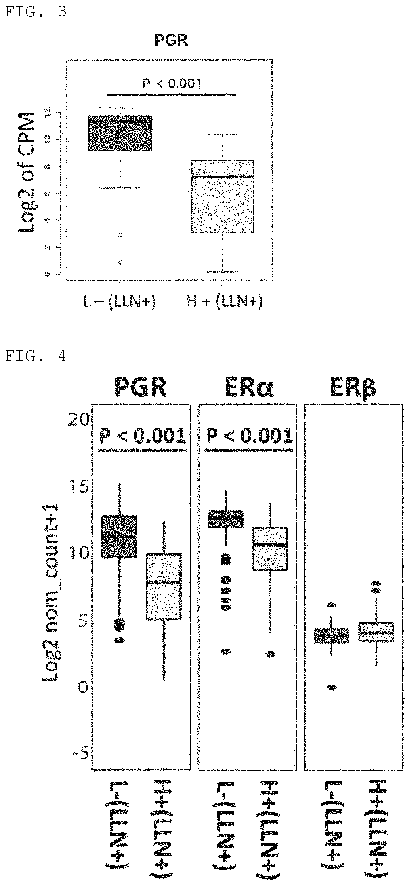

Fig. 3 is RNA expression levels of PGR in a lymph node metastasis negative group of a low to moderate risk group for recurrence (L-(LLN+))and a lymph node metastasis positive group of a high risk group + a low to moderate risk group recurrence (H+(LLN+))of endometrial cancer.
Fig. 4 is RNA expression levels (TCGA database) of PGR, ERa and ERβ in a lymph node metastasis negative group of a low to moderate recurrence risk group for recurrence (L-(LLN+)) and a lymph node metastasis positive group of a high risk group + a low to moderate risk group for recurrence (H+(LLN+)) of endometrial cancer.

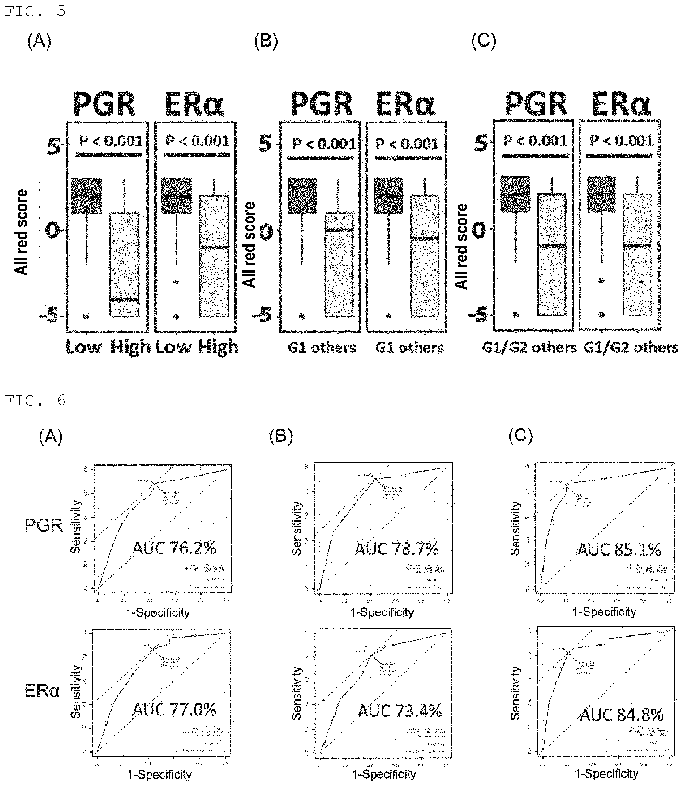

Fig. 5 is (A) expression levels of PGR and ERa in a low to moderate risk group for recurrence (Low) and a high risk group for recurrence (High)of endometrial cancer, (B) expression levels of PGR and ERa in endometrioid cancer G1 group (G1) and the other histological type groups (others) of endometrial cancer, and (C) expression levels of PGR and ERa in endometrioid cancer G1 or G2 group (G1/G2) and the other histological type groups (others) of endometrial cancer.
Fig. 6 is (A) discrimination accuracy of a low to moderate risk group for recurrence (Low) and a high risk group for recurrence (High) of endometrial cancer by PGR and ERα, (B) discrimination accuracy of endometrioid cancer G1 group (G1) and the other histological type groups (others) of endometrial cancer by PGR and ERα, and (C) discrimination accuracy of endometrioid cancer G1 or G2 group (G1/G2) and the other histological type groups (others) of endometrial cancer by PGR and ERα.

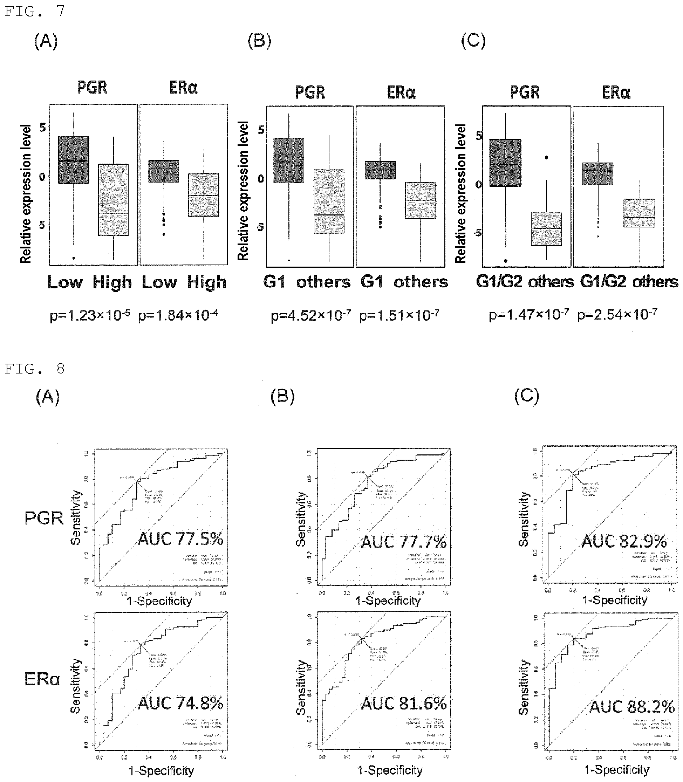

Fig. 7 is (A) expression levels of PGR and ERa in a low to moderate risk group for recurrence (Low) and a high risk group for recurrence (High) of endometrial cancer, (B) expression levels of PGR and ERa in endometrioid cancer G1 group (G1) and the other histological type groups (others) of endometrial cancer, and (C) expression levels of PGR and ERa in endometrioid cancer G1 or G2 group (G1/G2) and the other histological type groups (others) of endometrial cancer.
Fig. 8 is (A) discrimination accuracy of a low to moderate risk group for recurrence (Low) and a high risk group for recurrence (High) of endometrial cancer by PGR and ERα, (B) discrimination accuracy of endometrioid cancer G1 group (G1) and the other histological type groups (others) of endometrial cancer by PGR and ERα, and (C) discrimination accuracy of endometrioid cancer G1 or G2 group (G1/G2) and the other histological type groups (others) of endometrial cancer by PGR and ERα.

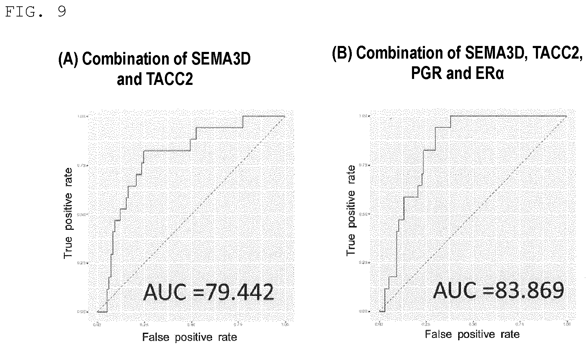

Fig. 9 is discrimination accuracy of lymph node metastatic potential by a combination of a marker for risk assessment for recurrence and a marker for assessment of lymph node metastatic potential of endometrial cancer. (A) SEMA3D and TACC2, (B) SEMA3D and TACC2+PGR and ERα.

### Description of Embodiments

In the present invention, endometrial cancer means cancer that occurs in the uterine corpus among uterine cancers. Endometrial cancer occurs in the endometrium, which is the epithelial tissue on the uterine cavity side, and is synonymous with uterine body cancer. Endometrial cancers include endometrioid adenocarcinoma, serous adenocarcinoma, clear cell adenocarcinoma, mucinous adenocarcinoma, and the like that differ in histological type. In endometrial cancer, the advanced stage is classified into I to IV according to the JSOG surgical staging classification (2011) and the FIGO (International Federation of Gynecology and Obstetrics) 2008 surgical staging classification on the basis of the size, spread, invasion, and metastasis of cancer. In addition, the endometrial cancer is classified into a well differentiated type (G1: grade 1), a moderately differentiated type (G2: grade 2), and a poorly differentiated type (G3: grade 3) depending on the malignancy of cancer, and the prognosis becomes worse in this order. In the present invention, endometrial cancer to be assessed for recurrence risk is not particularly limited by histological type, stage, or grade.

In the present invention, the recurrence risk of endometrial cancer refers to risk of recurring the cancer or recurring in the future. The recurrence risk of endometrial cancer is conventionally classified according to the tissue malignancy of cancer and the progress degree of cancer lesion. In the present invention, a low risk group for recurrence of endometrial cancer means a groupin which the risk is low. Preferably, the low risk means that endometrial cancer is G1 or G2 of endometrioid cancer with no or less than 1/2 muscle layer invasion. In addition, in the present invention, a moderate risk group for recurrence of endometrial cancer means a groupin which the risk is moderate. Preferably, the moderate risk means that endometrial cancer is G1 or G2 of endometrioid cancer with 1/2 or more muscle layer invasion without cervical invasion and extrauterine extension, G3 of endometrioid cancer with no or less than 1/2 muscle layer invasion, or the other histological types without muscle layer invasion. In addition, in the present invention, a high risk group for recurrence of endometrial cancer means a groupin which the risk is high. Preferably, the high risk means that endometrial cancer is not low risk or moderate risk endometrial cancer, specifically, the endometrial cancer is G1 or G2 of endometrioid cancer with cervical invasion or extrauterine extension, G3 of endometrioid cancer with 1/2 or more muscle layer invasion, with cervical invasion, or with extrauterine extension, or the other histological types with muscle layer invasion, with cervical invasion, or with extrauterine extension. The low to moderate risk group for recurrence includes the low risk group and the moderate risk group. In general, the rate of occurrence of lymph node metastasis in the low to moderate risk group for recurrence is about 10%, and lymph node metastasis occurs at high frequency in the high risk group (Fig. 1).

In the present invention, the assessment of recurrence risk of endometrial cancer means to assess, measure or detect the degree of recurrence risk of endometrial cancer, and specifically means to assess, measure or detect whether the recurrence risk of endometrial cancer is low risk or moderate risk (whether the endometrial cancer belongs to a low to moderate risk group) or whether the recurrence risk is high risk (whether the endometrial cancer belongs to a high risk group). In addition, the assessment of recurrence risk of endometrial cancer can also be rephrased as assessing, measuring or detecting the tissue malignancy of endometrial cancer, and specifically means to assess, measure or detect whether the tissue malignancy of endometrial cancer is G1 of endometrioid cancer or other (G2 or G3 of endometrioid cancer or other histological type), or G1 or G2 of endometrioid cancer or other (G3 of endometrioid cancer or other histological type).

In the present invention, lymph node metastasis in endometrial cancer means that endometrial cancer grows in lymph nodes in the pelvis, around the aorta, and the like.

In the present invention, the assessment of lymph node metastasis means to assess, measure or detect the presence or absence of metastasis of endometrial cancer to the lymph node, and the assessment of lymph node metastatic potential (lymph node metastatic property) means to assess, measure or detect whether or not endometrial cancer has the potential to metastasize to the lymph node and grow.

In the present invention, the assessment of prognosis means to predict the course and termination of endometrial cancer. Good or poor prognosis in endometrial cancer is closely related to cancer metastasis, and one of the main routes in cancer cell metastasis is lymph nodes. Therefore, the assessment of lymph node metastatic potential has a close correlation with the prognosis assessment of endometrial cancer, and when there is lymph node metastasis (potential), it can be assessed as poor prognosis, and when there is no lymph node metastasis (potential), it can be assessed as good prognosis.

The biological sample used in the present invention is an endometrial cancer tissue before or during surgery isolated from an endometrial cancer patient to be assessed. The biological sample is appropriately prepared and processed for measurement. For example, when the sample is subjected to measurement at the nucleic acid level, an RNA extract is prepared, and when the sample is subjected to measurement at the protein level, a protein extract is prepared, or the sample itself is used.

As a method for extracting RNA from the biological sample, any known method can be used. Specifically, Ambion RiboPure kit manufactured by Life Technologies Corporation, miRNeasy manufactured by Qiagen, and RNeasy manufactured by Qiagen can be exemplified, and among them, miRNeasy kit manufactured by Qiagen is suitably used.

In the present specification, the term "nucleic acid" means DNA or RNA. Further, the "DNA" includes not only double-stranded DNA but also single-stranded DNA such as a sense strand and an antisense strand constituting the double-stranded DNA. Therefore, DNA includes double-stranded genomic DNA, single-stranded cDNA, single-stranded DNA having a sequence complementary to the DNA, and the like. Furthermore, the "RNA" includes all of total RNA, mRNA, rRNA, and synthetic RNA.

As shown in the example described later, as a result of comprehensively analyzing the expression state of DNA of 100 bases or more downstream including the transcription start site on the genome using a CAGE (cap analysis gene expression) analysis method for endometrial cancer specimens, a transcription product of DNA consisting of the nucleotide sequences set forth in SEQ ID NOs: 1 to 6 (human genomic DNA consisting of the transcription start site and 100 bases continuous with the downstream thereof) was found to have a significant difference in expression level (transcriptional activity) between endometrial cancer with low or moderate risk of recurrence and no lymph node metastasis (negative) and other endometrial cancers, that is, endometrial cancer with high risk of recurrence and endometrial cancer with low or moderate risk of recurrence and lymph node metastasis (positive). Specifically, these transcription products were extracted by differential analysis on the transcriptional activity of RNA between a profile group derived from clinical specimens obtained from "endometrial cancer with low or moderate risk of recurrence and no lymph node metastasis" and a profile group derived from clinical specimens obtained from "other endometrial cancer" using R/Bioconductor edgeR package (Bioinformatics. 2010 Jan 1; 26(1): 139-40), with a threshold set to FDR (false discovery rate) of 5%.

In the present invention, the "transcription start site" refers to a region including a transcription start point. The transcription start point from a particular promoter is not limited to a single base and may be bases present at multiple positions downstream of the promoter on the genome. A region including the plurality of transcription start points is referred to as a transcription start site in the present specification. More specifically, the transcription start site is a region between a transcription start point located closest to the 5' side and a transcription start point located closest to the 3' side among the plurality of transcription start points. In each of the nucleotide sequences set forth in SEQ ID NOs: 1 to 6, the transcription start site is a base region forming a 5' end corresponding to a region whose both ends are defined by a base at position 1 (5' end) and a 101st base counted from the 3' end. In other words, each of the nucleotide sequences set forth in SEQ ID NOs: 1 to 6 shows a transcription start site and 100 bases following the transcription start point located closest to the 3' side in the transcription start site.

The DNA consisting of the nucleotide sequences set forth in SEQ ID NOs: 1 to 6 is a DNA consisting of the transcription start site of six variants of Progesterone receptor (PGR) and 100 bases continuous with the downstream thereof. As shown in Examples described later, these RNA transcriptional activities were significantly higher in a lymph node metastasis negative group of a low to moderate risk group for recurrence than in other groups, that is, a lymph node metastasis positive group of a high risk group and a low to moderate risk group for recurrence (Fig. 2). In addition, even when the six variants were grouped without distinction, the RNA transcriptional activities were significantly higher in a lymph node metastasis negative group of a low to moderate risk group for recurrence than in the other groups, that is, a lymph node metastasis positive group of a high risk group and a low to moderate risk group for recurrence (Fig. 3).

As shown in Examples described later, as a result of acquiring clinical records and RNA-seq analysis results in TCGA (The Cancer Genome Atlas) database from GDC (Genomic Data Commons) Data Portal (http://gdc.nci.nih.gov/) of the National Institutes of Health, and confirming the expression level of PGR, the expression level of PGR was similarly significantly higher in the lymph node metastasis negative group of a low to moderate risk group for recurrence than the other groups, that is, the lymph node metastasis positive group of a high risk group and a low to moderate risk group for recurrence (Fig. 4). In addition, the expression level of estrogen receptor α (ERα) was significantly higher in the lymph node metastasis negative group of a low to moderate risk group for recurrence than in the other groups, that is, the lymph node metastasis positive group of a high risk group and a low to moderate risk group for recurrence (Fig. 4).

Furthermore, as shown in Examples described later, as a result of verifying the expression levels of PGR and ERa for endometrial cancer specimens, the low to moderate risk group for recurrence was significantly higher than the high risk group for recurrence (Figs. 5(A) and 7(A)), and both the sensitivity and specificity showed sufficient discernment (Figs. 6(A) and 8(A)). In addition, the expression levels of PGR and ERa were significantly higher in the group in which the tissue malignancy of endometrial cancer is G1 of endometrioid cancer than those in the other groups (Figs. 5(B) and 7(B)), and both the sensitivity and specificity showed sufficient discernment (Figs. 6(B) and 8(B)). Moreover, the expression levels of PGR and ERa were significantly higher in the group in which the tissue malignancy is G1 or G2 of endometrioid cancer than those in the other groups (Figs. 5(C) and 7(C)), and both the sensitivity and specificity showed sufficient discernment (Figs. 6(C) and 8(C)).

Therefore, PGR and ERa can be markers for assessing recurrence risk of endometrial cancer. The expression level of these markers increases when the recurrence risk of endometrial cancer is low risk or moderate risk and decreases when the recurrence risk is high risk. In addition, the expression level of the markers increases when the tissue malignancy of endometrial cancer is G1 of endometrioid cancer and decreases when the tissue malignancy is the other. Moreover, expression level of the markers increases when the tissue malignancy of endometrial cancer is G1 or G2 of endometrioid cancer and decreases when the tissue malignancy is the other. These markers may be used alone, or two markers may be used in combination, and it is preferable to use two markers in combination.

The genes and proteins of PGR and ERa are registered in NCBI as follows.
PGR Gene: NM_000926.4 (SEQ ID NO: 7)
PGR Protein: NP_000917.3 (SEQ ID NO: 8)
ERa Gene: NM_000125.3 (SEQ ID NO: 9)
ERa Protein: NP_000116.2 (SEQ ID NO: 10)

In the present invention, the method for measuring the expression level of at least one selected from the group consisting of PGR and ERa (hereinafter, these are referred to as "markers") is not particularly limited as long as it is a method capable of confirming a marker in a biological sample. That is, any method capable of detecting or quantifying the expression level of the marker may be used, and suitable examples thereof include detection or quantification of the marker at the nucleic acid level, specifically, detection or quantification of a transcription product (mRNA), and detection or quantification at the protein level, specifically, detection or quantification of a translation product (protein). Among them, it is preferable to detect or quantify a translation product (protein).

The detection or quantitative method at the nucleic acid level can be selected from nucleic acid amplification methods typified by PCR using a DNA hybridizing to a nucleic acid derived from a marker gene as a primer, RT-PCR, SmartAmp, LAMP, or the like, hybridization using a nucleic acid hybridizing to a nucleic acid derived from a marker gene as a probe (DNA chip, DNA microarray, dot blot hybridization, slot blot hybridization, Northern blot hybridization, and the like), a method for determining a nucleotide sequence, or a method combining these methods.

Here, a probe or a primer used for measurement, that is, thoses corresponding to a primer for specifically recognizing and amplifying a nucleic acid derived from a marker gene, or a probe for specifically detecting a nucleic acid derived from a marker gene can be designed based on the nucleotide sequence set forth in SEQ ID NO: 7 or SEQ ID NO: 9. Here, the phrase "specifically recognizing" means that it is possible to detect substantially only a nucleic acid derived from a marker gene in, for example, Northern blotting, and it is possible to determine that the detection product or the product is a nucleic acid derived from the marker gene so that substantially only the nucleic acid is generated in, for example, RT-PCR.

Specifically, an oligonucleotide containing a certain number of nucleotides complementary to a DNA consisting of the nucleotide sequence set forth in SEQ ID NO: 7 or SEQ ID NO: 9 or a complementary strand thereof can be used. Here, the term "complementary strand" refers to the other strand with respect to one strand of double-stranded DNA consisting of A: T (U in the case of RNA) and G: C base pairs. In addition, the term "complementary" is not limited to a case of a completely complementary sequence in a region with the certain number of consecutive nucleotides, and may have preferably 80% or more, more preferably 90% or more, and further preferably 95% or more identity of the nucleotide sequence. The identity of the nucleotide sequence can be determined, for example, using homology calculation algorithm NCBI BLAST, under the conditions of expected value = 10; gap allowed; filtering = ON; match score = 1; mismatch score = -3.

For use as a primer, the oligonucleotide may achieve specific annealing and strand extension, and examples thereof usually include oligonucleotides having a strand length of 10 or more bases, preferably 15 or more bases, and more preferably 20 or more bases, and 100 or less bases, preferably 50 or less bases, and more preferably 35 or less bases.

Also, for use as a probe, the oligonucleotide may achieve specific hybridization, and an oligonucleotide is used which has at least a portion or the whole of the sequence of DNA (or a complementary strand thereof) consisting of the nucleotide sequence set forth in SEQ ID NO: 7 or SEQ ID NO: 9, and has a strand length of, for example, 10 or more bases, and preferably 15 or more bases, and for example, 100 or less bases, preferably 50 or less bases, and more preferably 25 or less bases.

Specific hybridization means to hybridize substantially only to a nucleic acid of interest, and examples thereof include hybridization under stringent conditions. Here, examples of the stringent conditions can usually include washing conditions of "1 × SSC, 0.1% SDS, and 37°C", examples of the more stringent hybridization conditions can include conditions of "0.5 × SSC, 0.1% SDS, and 42°C", and examples of the much more stringent hybridization conditions can include conditions of "0.1 × SSC, 0.1% SDS, and 65°C". The hybridization conditions are described in J. Sambrook et al., Molecular Cloning: A Laboratory Manual, Third Edition, Cold Spring Harbor Laboratory Press (2001), and the like.

The "oligonucleotide" can be DNA or RNA, and may be synthesized or natural. As the probe used for hybridization, a labeled probe is usually used. Examples of a labeling agent include radioisotopes (examples: ³²P, ³³P, ³⁵S, ¹²⁵I, ¹³¹I, ³H, ¹⁴C, and the like), enzymes (examples: β-galactosidase, β-glucosidase, alkaline phosphatase, peroxidase, malate dehydrogenase, and the like), fluorescent substances (examples: fluorescamine, fluorescein isothiocyanate, and the like), luminescent substances (examples: luminol, luminol derivatives, luciferin, lucigenin, and the like), and the like.

For example, in the case of using Northern blot hybridization, probe DNA is first labeled with a radioisotope, a fluorescent material, or the like, and subsequently, the obtained labeled DNA is hybridized to biological sample-derived RNA transferred to a nylon membrane or the like according to a conventional method. Thereafter, the formed duplex of the labeled DNA and the RNA can be measured by detecting a signal derived from the labeled product.

In addition, in the case of using the RT-PCR, cDNA is first prepared from biological sample-derived RNA according to a conventional method, and a pair of primers (a positive strand which binds to the cDNA (- strand), a reverse strand which binds to a + strand) is prepared using the cDNA as a template so as to be able to amplify an expression product (transcription product) of a target marker, and the pair of primers is hybridized thereto. Thereafter, PCR is performed according to a conventional method, and the obtained amplified double-stranded DNA is detected. For the detection of the amplified double-stranded DNA, a method of detecting labeled double-stranded DNA produced by performing the PCR using primers labeled in advance with RI, a fluorescent substance, or the like can be used.

In addition, in the case of measuring the expression level of mRNA in a specimen by use of a DNA microarray, an array in which at least one nucleic acid (cDNA or DNA) derived from a marker gene is immobilized on a support is used, and the mRNA expression level can be measured by binding labeled cDNA or cRNA prepared from mRNA onto the microarray, and detecting the label on the microarray.

The nucleic acid immobilized on the array may be a nucleic acid which hybridizes under stringent conditions, and may be, for example, a nucleic acid having the entire sequence of the marker gene or a nucleic acid consisting of a partial sequence. Here, examples of the "partial sequence" include nucleic acids consisting of at least 15 to 25 bases.

In the measurement described above, in the case of comparing the expression variation of the marker, the measured value such as the expression level can be corrected in advance as a relative value with respect to a gene whose expression level is relatively stable. The correction makes it possible to more accurately compare the expression variations of the markers in a plurality of independent biological samples. The correction of the measured value can be performed by correcting the expression level of the marker gene using a gene whose expression level does not greatly vary depending on the degree of recurrence risk of endometrial cancer as an internal standard. Here, as a gene whose expression level is stable, GAPDH which is a housekeeping gene is generally used, but in the present invention, it was confirmed that SUDS3 gene (NM_022491) exhibits a more stable expression level in endometrial cancer cells, and the SUDS3 gene was used as an internal standard.

In the present invention, the measurement of the expression level of a marker also includes measuring the expression level of a homolog of the marker whose biological activity is equivalent to that of the marker.

Examples of such a homolog gene include a polynucleotide consisting of a nucleotide sequence that hybridizes to a complementary sequence of the nucleotide sequence set forth in SEQ ID NO: 7 or SEQ ID NO: 9 under stringent conditions, and encoding a protein having a biological activity equivalent to that of a protein consisting of the amino acid sequence set forth in SEQ ID NO: 8 or SEQ ID NO: 10.

Here, examples of the stringent conditions can include "1 × SSC, 0.1% SDS, and 37°C", more stringent condition inclyde "0.5 × SSC, 0.1% SDS, 42°C", and further much more stringent condition include "0.1 × SSC, 0.1% SDS, 65°C" as described above. Specific examples of such a polynucleotide include a polynucleotide consisting of a nucleotide sequence having 80% or more, preferably 90% or more, and more preferably 95% or more identity to the nucleotide sequence set forth in SEQ ID NO: 7 or SEQ ID NO: 9. The identity of the nucleotide sequence can be determined by an algorithm such as the BLAST.

For detection or quantification of a marker at the protein level, a method for immunologically measuring using an antibody that specifically binds to a marker protein (anti-PGR antibody, anti-ERα antibody) is simple and suitable. Examples of the immunological measurement method include enzyme immunoassay (ELISA), radioimmunoassay, immunohistochemistry staining, Western blot, immunoprecipitation, immunofluorescence, flow cytometry, and the like.

For example, according to Western blotting, after the above-described antibody is used as a primary antibody, the primary antibody is labeled using an antibody that binds to the primary antibody labeled with a radioisotope, a fluorescent material, an enzyme, or the like as a secondary antibody, and signals derived from these labeling substances are measured with a radiation measuring instrument, a fluorescence detector, or the like.

In addition, in the case of using an immunohistochemical staining method, it is preferable that a biological sample isolated from a patient is formalinfixed by a conventional method, then embedded in paraffin, sliced into a tissue piece, and attached to a slide glass, and the attached sample is used as a section sample. After the above-described antibody is used as the primary antibody, an enzyme-labeled antibody such as alkaline phosphatase or peroxidase can be used as the secondary antibody, but it is preferable to perform highly sensitive detection using a three-step method such as an ABC method or an LSAB method, an EnVision detection system of DAKO, an automatic staining system VENTANA BenchMark ULTRA of Ventana, or the like.

Here, the stained cells can be assessed based on known criteria, and examples of such criteria include J score, Allred score, and the like. In the case of using J score, the number of stained cells is assessed in four stages of 0: negative, 1: positive cell occupancy of less than 1%, 2: positive cell occupancy of 1% or more and less than 10%, and 3: positive cell occupancy of 10% or more. In the case of using Allred score (Mod Pathol 11: 155-168, 1998), the staining occupancy (Proportion score: PS) of stained cells is determined in six stages of 0: no staining, 1: less than 1/100, 2: 1/100 or more and less than 1/10, 3: 1/10 or more and less than 1/3, 4: 1/3 or more and less than 2/3, and 5: 2/3 or more, and the staining intensity (Intensity score: IS) is determined in four stages of 0: negative, 1: weak staining, 2: moderate staining, and 3: strong staining. The total of these two values (Total score: TS) is classified into nine stages. Among them, the Allred score is preferably used.

The anti-PGR antibody and the anti-ERα antibody may be a polyclonal antibody or a monoclonal antibody. In addition, it is also possible to use a fragment of an antibody which is a part (partial fragment) of an antibody or a peptide containing a part of an antibody and retains a binding action of the antibody to an antigen (marker protein). Examples of the antibody fragment include F(ab')₂, Fab', Fab, single-strand Fv (scFv), and the like.

These antibodies can be produced according to a known method. Specifically, the polyclonal antibody can be obtained by using a protein which has been expressed in *E*. *coli* or the like and purified according to a conventional method, or synthesizing a partial polypeptide of the protein according to a conventional method, and immunizing a non-human animal such as a house rabbit therewith, followed by obtainment from the serum of the immunized animal according to a conventional method.

On the other hand, the monoclonal antibody can be obtained from hybridoma cells prepared by immunizing a non-human animal such as a mouse with a protein which has been expressed in *E. coli* or the like and purified according to a conventional method or a partial polypeptode of the protein, and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108(7)).

A fragment of an antibody can be obtained by enzymatically treating the obtained antibody or using sequence information of the obtained antibody.

Alternatively, a commercially available antibody may be used as the anti-PGR antibody and the anti-ERα antibody. Such antibodies include anti-PGR antibodies (Ventana CONFIRM PR (1E2), product number 790-2223, manufactured by Ventana), anti-ERα antibodies (Ventana CONFIRM ER (SP1), product number 790-4324, manufactured by Ventana), and the like.

Thus, the expression level of a marker in a biological sample derived from a cancer tissue isolated from an endometrial cancer patient is measured, and the recurrence risk is assessed based on the expression level. Specifically, it is assessed by comparing the expression level of the detected marker with a control level.

Here, examples of the "control level" include an expression level of a marker in an endometrial cancer tissue isolated from an endometrial cancer patient with low risk or moderate risk (hereinafter, also referred to as low to moderate risk) of recurrence or a normal tissue isolated from an endometrial cancer patient, or an expression level of a marker in a healthy individual group who has not developed endometrial cancer.

For example, when the expression level of PGR in the cancer tissue of the subject patient is close to the expression level in an endometrial cancer tissue isolated from an endometrial cancer patient with low to moderate risk of recurrence, a normal tissue thereof, or a tissue derived from a healthy individual, falls within the range of the expression level, or is significantly higher than the expression level, the endometrial cancer of the patient can be assessed as having low to moderate risk of recurrence. On the other hand, when the expression level of PGR in the cancer tissue of the subject patient is significantly lower than the expression level in an endometrial cancer tissue isolated from an endometrial cancer patient with low to moderate risk of recurrence, a normal tissue thereof, or a tissue derived from a healthy individual, the endometrial cancer of the patient can be assessed as having high risk of recurrence.

In addition, when the expression level of ERa in the cancer tissue of the subject patient is close to the expression level in an endometrial cancer tissue isolated from an endometrial cancer patient with low to moderate risk of recurrence, a normal tissue thereof, or a tissue derived from a healthy individual, falls within the range of the expression level, or is significantly higher than the expression level, the endometrial cancer of the patient can be assessed as having low to moderate risk of recurrence. On the other hand, when the expression level of ERa in the cancer tissue of the subject patient is significantly lower than the expression level in an endometrial cancer tissue isolated from an endometrial cancer patient with low to moderate risk of recurrence, a normal tissue thereof, or a tissue derived from a healthy individual, the endometrial cancer of the patient can be assessed as having high risk of recurrence.

As another example, examples of the control level include an expression level of a marker in an endometrioid cancer tissue isolated from an endometrial cancer patient of G1 of endometrioid cancer.

For example, when the expression level of PGR in the cancer tissue of the subject patient is close to the expression level in an endometrioid cancer tissue isolated from an endometrial cancer patient of G1 of endometrioid cancer, falls within the range of the expression level, or is significantly higher than the expression level, the endometrial cancer of the patient can be assessed as G1 of endometrioid cancer. On the other hand, when the expression level of PGR in the cancer tissue of the subject patient is significantly lower than the expression level in an endometrioid cancer tissue isolated from an endometrial cancer patient of G1 of endometrioid cancer, the endometrial cancer of the patient can be assessed as other endometrial cancer, that is, endometrial cancer with higher tissue malignancy than G1 of endometrioid cancer.

In addition, when the expression level of ERa in the cancer tissue of the subject patient is close to the expression level in an endometrioid cancer tissue isolated from an endometrial cancer patient of G1 of endometrioid cancer, falls within the range of the expression level, or is significantly higher than the expression level, the endometrial cancer of the patient can be assessed as G1 of endometrioid cancer. On the other hand, when the expression level of ERa in the cancer tissue of the subject patient is significantly lower than the expression level in an endometrioid cancer tissue isolated from an endometrial cancer patient of G1 of endometrioid cancer, the endometrial cancer of the patient can be assessed as other endometrial cancer, that is, endometrial cancer with higher tissue malignancy than G1 of endometrioid cancer.

As yet another example, examples of the control level include an expression level of a marker in an endometrioid cancer tissue isolated from an endometrial cancer patient of G1 or G2 of endometrioid cancer.

For example, when the expression level of PGR in the cancer tissue of the subject patient is close to the expression level in an endometrioid cancer tissue isolated from an endometrial cancer patient of G1 or G2 of endometrioid cancer, falls within the range of the expression level, or is significantly higher than the expression level, the endometrial cancer of the patient can be assessed as G1 or G2 of endometrioid cancer. On the other hand, when the expression level of PGR in the cancer tissue of the subject patient is significantly lower than the expression level in an endometrioid cancer tissue isolated from an endometrial cancer patient of G1 or G2 of endometrioid cancer, the endometrial cancer of the patient can be assessed as other endometrial cancer, that is, endometrial cancer with higher tissue malignancy than G1 and G2 of endometrioid cancer.

In addition, when the expression level of ERa in the cancer tissue of the subject patient is close to the expression level in an endometrioid cancer tissue isolated from an endometrial cancer patient of G1 or G2 of endometrioid cancer, falls within the range of the expression level, or is significantly higher than the expression level, the endometrial cancer of the patient can be assessed as G1 or G2 of endometrioid cancer. On the other hand, when the expression level of ERa in the cancer tissue of the subject patient is significantly lower than the expression level in an endometrioid cancer tissue isolated from an endometrial cancer patient of G1 or G2 of endometrioid cancer, the endometrial cancer of the patient can be assessed as other endometrial cancer, that is, endometrial cancer with higher tissue malignancy than G1 and G2 of endometrioid cancer.

The recurrence risk of endometrial cancer in the present invention can also be assessed by increasing/decreasing the expression level of a marker. In this case, as the control level, for example, a standard value (threshold level) is set based on the expression level of a marker derived from a normal tissue, an endometrial cancer tissue isolated from an endometrial cancer patient with low to moderate risk of recurrence, or a tissue of a healthy individual, and the expression level of the marker in a patient-derived biological sample can be compared with the standard value (for example, a range of ±2 S.D. is an allowable range).

For example, when the expression level of PGR in a patient-derived biological sample is higher than the threshold level, the endometrial cancer of the patient can be assessed as having low to moderate risk of recurrence. On the other hand, when the expression level of PGR in a patient-derived biological sample is lower than the threshold level, the endometrial cancer of the patient can be assessed as having high risk of recurrence.

In addition, when the expression level of ERa in a patient-derived biological sample is higher than the threshold level, the endometrial cancer of the patient can be assessed as having low to moderate risk of recurrence. On the other hand, when the expression level of ERa in a patient-derived biological sample is lower than the threshold level, the endometrial cancer of the patient can be assessed as having high risk of recurrence.

In another example, as the control level, for example, a standard value (threshold level) is set based on the expression level of a marker in an endometrioid cancer tissue isolated from an endometrial cancer patient of G1 of endometrioid cancer, and the expression level of the marker in a patient-derived biological sample can be compared with the standard value (for example, a range of ±2 S.D. is an allowable range).

For example, when the expression level of PGR in a patient-derived biological sample is higher than the threshold level, the endometrial cancer of the patient can be assessed as G1 of endometrioid cancer. On the other hand, when the expression level of PGR in a patient-derived biological sample is lower than the threshold level, the endometrial cancer of the patient can be assessed as endometrial cancer of other histological type, that is, endometrial cancer with higher tissue malignancy than G1 of endometrioid cancer.

In addition, when the expression level of ERa in a patient-derived biological sample is higher than the threshold level, the endometrial cancer of the patient can be assessed as G1 of endometrioid cancer. On the other hand, when the expression level of ERa in a patient-derived biological sample is lower than the threshold level, the endometrial cancer of the patient can be assessed as endometrial cancer of other histological type, that is, endometrial cancer with higher tissue malignancy than G1 of endometrioid cancer.

In still another example, as the control level, for example, a standard value (threshold level) is set based on the expression level of a marker in an endometrioid cancer tissue isolated from an endometrial cancer patient of G1 or G2 of endometrioid cancer, and the expression level of the marker in a patient-derived biological sample can be compared with the standard value (for example, a range of ±2 S.D. is an allowable range).

For example, when the expression level of PGR in a patient-derived biological sample is higher than the threshold level, the endometrial cancer of the patient can be assessed as G1 or G2 of endometrioid cancer. On the other hand, when the expression level of PGR in a patient-derived biological sample is lower than the threshold level, the endometrial cancer of the patient can be assessed as other endometrial cancer, that is, endometrial cancer with higher tissue malignancy than G1 and G2 of endometrioid cancer.

In addition, when the expression level of ERa in a patient-derived biological sample is higher than the threshold level, the endometrial cancer of the patient can be assessed as G1 or G2 of endometrioid cancer. On the other hand, when the expression level of ERa in a patient-derived biological sample is lower than the threshold level, the endometrial cancer of the patient can be assessed as other endometrial cancer, that is, endometrial cancer with higher tissue malignancy than G1 and G2 of endometrioid cancer.

According to the method of the present invention, the recurrence risk of endometrial cancer is determined on the basis of information provided in combination with other methods (CT, MRI, PET-CT, and the like) as necessary. The treatment policy based on the recurrence risk is left to the judgment of the doctor, but depending on the determined risk, for example, it is possible to improve life prognosis of the patient by early treatment (surgical treatment, chemotherapy, radiotherapy, or the like) and to intervene prophylactically for avoiding recurrence.

Here, as described above, the present inventors have already found that Semaphorin 3D (SEMA3D) and Transforming acidic coiled-coil-containing protein 2 (TACC2) can be markers for assessing lymph node metastasis or lymph node metastatic potential or prognosis of endometrial cancer (Patent Literatures 1 and 2). Such a marker enables highly accurate assessment particularly for endometrial cancer patients with low to moderate risk of recurrence. The specimen determined to have a low to moderate risk of recurrence of endometrial cancer by the method of the present invention is suitable as a target for assessing lymph node metastasis or lymph node metastatic potential or prognosis of endometrial cancer by measuring the expression level of at least one selected from the group consisting of SEMA3D and TACC2. Therefore, the method of the present invention may be a method of selecting an endometrial cancer specimen with low to moderate risk of recurrence.

As described above, at least one marker selected from the group consisting of PGR and ERa of the present invention is useful as a companion marker for selecting an endometrial cancer specimen with low to moderate risk of recurrence suitable in assessing lymph node metastasis or lymph node metastatic potential or prognosis of endometrial cancer based on the expression level of at least one selected from the group consisting of SEMA3D and TACC2. In other words, it is possible to select an endometrial cancer specimen with low to moderate risk of recurrence based on the expression level of at least one selected from the group consisting of PGR and ERα. The selected endometrial cancer specimen with low to moderate risk of recurrence can be subjected to assessment of lymph node metastasis or lymph node metastatic potential or prognosis of endometrial cancer by measuring the expression level of at least one selected from the group consisting of SEMA3D and TACC2.

SEMA3D has a gene registered as NM_152754.3 (SEQ ID NO: 11) and a protein registered as NP_689967.2 (SEQ ID NO: 12) in NCBI.

On the other hand, TACC2 has a protein registered in UniProtKB/Swiss-Prot as TACC2#HUMAN (095359), but in the present invention, a TACC2 isoform present in the TACC2 gene locus can also be used. Here, examples of the TACC2 isoform include a gene consisting of a nucleotide sequence set forth in SEQ ID NO: 13 (TACC2 isoform L) or 14 (TACC2 isoform S) having chromosome 10 (chr10), a start position 123779316, and an end position 123779341 of the human reference genome (GRCh37) as a transcription start site (TSS), and any one or a part of UniProtKB/Swiss-Prot: TACC2#HUMAN (095359, SEQ ID NO: 15), E9PBC6#HUMAN (E9PBC6, SEQ ID NO: 16), and E7EMZ9#HUMAN (E7EMZ9, SEQ ID NO: 17), which are proteins encoded therefrom.

SEMA3D is a marker having a significantly higher expression level when there is no lymph node metastasis as compared with that when there is lymph node metastasis in endometrial cancer (Patent Literature 2), and TACC2 is a marker having a significantly higher expression level when there is lymph node metastasis as compared with that when there is no lymph node metastasis in endometrial cancer (Patent Literatures 1 and 2). In addition, the relative expression level of SEMA3D and TACC2 <SEMA3D-TACC2> is higher when there is no lymph node metastasis as compared with that when there is lymph node metastasis in endometrial cancer. When SEMA3D and TACC2 are combined, sensitivity and specificity are superior to those when each is used alone, and thus the combination is extremely useful as a marker for assessing lymph node metastasis or lymph node metastatic potential or prognosis of endometrial cancer.

In the present invention, the method for measuring the expression level of at least one selected from the group consisting of SEMA3D and TACC2 is similar to the case of PGR and ERa described above.

Both of the expression level of at least one marker selected from the group consisting of PGR and ERa in a biological sample derived from a cancer tissue isolated from an endometrial cancer patient and the expression level of at least one marker selected from the group consisting of SEMA3D and TACC2 in the biological sample may be measured, or only when the expression level of at least one marker selected from the group consisting of PGR and ERa may be measured and it is determined that the recurrence risk is low or moderate, the expression level of at least one marker selected from the group consisting of SEMA3D and TACC2 may be measured .

Thus, the expression level of at least one marker selected from the group consisting of SEMA3D and TACC2 in a biological sample derived from a cancer tissue isolated from an endometrial cancer patient is measured, and the presence or absence of lymph node metastasis or lymph node metastatic potential or poor prognosis is assessed based on the expression level. Specifically, it is assessed by comparing the expression level of the detected marker with a control level.

Here, examples of the "control level" include an expression level of a marker in an endometrial cancer tissue isolated from an endometrial cancer patient without lymph node metastasis or a normal tissue isolated from an endometrial cancer patient, or an expression level of a marker in a healthy individual group who has not developed endometrial cancer.

For example, when the expression level of SEMA3D in the cancer tissue of the subject patient is close to the expression level in an endometrial cancer tissue isolated from an endometrial cancer patient without lymph node metastasis, a normal tissue thereof, or a tissue derived from a healthy individual, falls within the range of the expression level, or is significantly higher than the expression level, it can be assessed that the endometrial cancer of the patient has no lymph node metastasis or has low lymph node metastatic potential, or has a good prognosis.

In addition, when the expression level of TACC2 in the cancer tissue of the subject patient is close to the expression level in an endometrial cancer tissue isolated from an endometrial cancer patient without lymph node metastasis, a normal tissue thereof, or a tissue derived from a healthy individual, falls within the range of the expression level, or is significantly lower than the expression level, it can be assessed that the endometrial cancer of the patient has no lymph node metastasis, has low lymph node metastatic potential, or has a good prognosis.

In addition, when the relative expression level of SEMA3D with respect to the expression level of TACC2 in the cancer tissue of the subject patient is close to the expression level in an endometrial cancer tissue isolated from an endometrial cancer patient without lymph node metastasis, a normal tissue thereof, or a tissue derived from a healthy individual, falls within the range of the expression level, or is significantly higher than the expression level, it can be assessed that the endometrial cancer of the patient has no lymph node metastasis or has low lymph node metastatic potential, or has a good prognosis.

Moreover, the lymph node metastasis or lymph node metastatic potential of endometrial cancer in the present invention can also be assessed by increasing/decreasing the expression level of a marker. In this case, as the control level, for example, a standard value (threshold level) is set based on the expression level of a marker derived from a normal tissue, an endometrial cancer tissue isolated from an endometrial cancer patient without lymph node metastasis, or a tissue of a healthy individual, and the expression level of the marker in a patient-derived biological sample can be compared with the standard value (for example, a range of ±2 S.D. is an allowable range).

For example, when the expression level of SEMA3D in a patient-derived biological sample is higher than the threshold level, it can be assessed that the endometrial cancer of the patient has no lymph node metastasis or has low lymph node metastatic potential, or has a good prognosis.

Further, when the expression level of TACC2 in a patient-derived biological sample is lower than the threshold level, it can be assessed that the endometrial cancer of the patient has no lymph node metastasis or has low lymph node metastatic potential, or has a good prognosis.

Furthermore, when the relative expression level of SEMA3D with respect to the expression level of TACC2 in a patient-derived biological sample is higher than the threshold level, it can be assessed that the endometrial cancer of the patient has no lymph node metastasis or has low lymph node metastatic potential, or has a good prognosis.

From the above, according to the method of the present invention, the possibility of lymph node metastasis or lymph node metastatic potential or prognosis is determined on the basis of information provided in combination with other methods (CT, MRI, PET-CT, and the like) as necessary. The criteria for performing lymph node biopsy and lymph node dissection are left to the judgment of the doctor, but when it has been determined that there is a possibility of lymph node metastasis or the lymph node metastatic potential is high, for example, the lymph node dissection can be performed. On the other hand, when it has been determined that there is no possibility of lymph node metastasis, the lymph node metastatic potential is low, or the prognosis is good, it is considered not necessary to perform lymph node dissection.

The test kit for assessing recurrence risk of endometrial cancer and the test kit for assessing lymph node metastasis or lymph node metastatic potential or prognosis of endometrial cancer of the present invention include a test reagent or a measuring device for measuring the expression level of a marker in a biological sample isolated from a patient. Specific examples thereof include a reagent for nucleic acid amplification or hybridization containing an oligonucleotide that specifically binds (hybridizes) to a transcription product (mRNA) of a marker, a reagent for immunological measurement containing an antibody that recognizes a translation product (protein) of a marker of the present invention, an immunoassay device on which the antibody is immobilized, and the like. The oligonucleotide, antibody and the like included in the kit can be obtained by a known method as described above.

Also, in addition to the antibody and the nucleic acid, the test kit can include a labeling reagent, a buffer, a chromogenic substrate, a secondary antibody, a blocking agent, an instrument necessary for a test, a control, and the like.

The device for assessing recurrence risk of endometrial cancer and the device for assessing lymph node metastasis or lymph node metastatic potential or prognosis of endometrial cancer of the present invention include a means for measuring the expression level of a marker, a means for assessing or determining acquired data, and a means for outputting the result of the assessment or determination, and can be arbitrarily configured using known means.

### Examples

Next, the present invention will be described more in detail by way of examples thereof. It should be noted, however, that the technical scope of the present invention is not limited to these examples.

### Specimen

115 Cases of endometrial cancer patients from whom comprehensive consent was obtained before surgery were targeted. Here, sarcoma-like cases and cases receiving preoperative adjuvant chemotherapy were excluded. Clinical background of cases including the stage based on the surgical staging classification by FIGO2008, histological type, grade, the presence or absence of lymph node metastasis, and the like is shown in Table 1. Several specimens of 5 mm square were collected from a main lesion at the time of primary lesion extraction in each case. The collected specimen was frozen in liquid nitrogen immediately after extraction and stored at -80°C until RNA extraction. In addition, a specimen of the main lesion was collected at the time of primary lesion extraction, fixed with formalin by a conventional method, and embedded in paraffin.

**[Table 1]**

| | | Number of cases 115 | % |
|---|---|---|---|
| Average age (range) | | 56 (31-85) | - |
| Average number of pregnancies (range) | | 1 (0-5) | - |
| Average number of childbirth (range) | | 0 (0-4) | - |
| Menopause | | 64 | 55.7 |
| Body mass index (BMI) > 30 | | 19 | 16.5 |
| Diabetes | | 17 | 14.8 |
| Hypertension | | 23 | 20.0 |
| Smoker | | 10 | 8.7 |
| Medical history of breast cancer | | 11 | 9.6 |
| Medical history of other cancer | | 8 | 7.0 |
| Histological type | | | |
| | Endometrioid carcinoma | 104 | 90.4 |
| | Non-endometrioid carcinoma | 11 | 9.6 |
| FIGO stage | | | |
| | I | 84 | 73.0 |
| | II | 6 | 5.2 |
| | III | 17 | 14.8 |
| | IV | 8 | 7.0 |
| Grade | | | |
| | 1 | 77 | 67.0 |
| | 2 | 29 | 25.2 |
| | 3 | 9 | 7.8 |
| Lymph node metastasis | | | |
| | Present | 97 | 84.3 |
| | Absent | 18 | 15.7 |

Example 1 Extraction and verification of transcription start site enabling determination of recurrence risk of endometrial cancer

### (1) Specimen

40 Cases out of 115 cases of endometrial cancer in Table 1 were grouped on the basis of the tissue malignancy of cancer and the recurrence risk based on the progression degree of cancer lesion. Specifically, cases of endometrioid carcinoma G1 or G2 with no or less than 1/2 muscle layer invasion were defined as a low risk group for recurrence, cases of endometrioid carcinoma G1 or G2 with 1/2 or more muscle layer invasion without cervical invasion and extrauterine extension, cases of endometrioid carcinoma G3 with no or less than 1/2 muscle layer invasion, and cases of other histological types without muscle layer invasion were defined as a moderate risk group for recurrence, and the other cases were defined as a high risk group for recurrence. As a result, the cases were divided into a low to moderate risk group of 29 cases (8 cases with lymph node metastasis and 21 cases without lymph node metastasis) and a high risk group of 11 cases (7 cases with lymph node metastasis, 4 cases without lymph node metastasis) (Table 2). In addition, those obtained by excluding cases with lymph node metastasis from the low to moderate risk group for recurrence of endometrial cancer in Table 2 were defined as a metastasis-negative group of the low to moderate risk group for recurrence of endometrial cancer, and those obtained by adding cases with lymph node metastasis of the low to moderate risk group for recurrence to the high risk group for recurrence were defined as a metastasis-positive group of the high risk group and the low to moderate risk group for recurrence of endometrial cancer, and the following tests were performed.

**[Table 2]**

| | | Recurrence risk | |
|---|---|---|---|
| Factor | | Low to moderate risk (%) | High risk (%) |
| Case | | 29 | 11 |
| Age (average - range) | | 51 (37-70) | 61 (45-76) |
| Menopause | | 11 (37.9) | 6 (54.5) |
| Histological type | | | |
| | Endometrioid carcinoma G1 | 26 (89.7) | 4 (36.4) |
| | Endometrioid carcinoma G2 | 3 (10.3) | 2 (18.2) |
| | Endometrioid carcinoma G3 | 0 (0.0) | 3 (27.2) |
| | Other histological type | 0 (0.0) | 2* (18.2) |
| Tumor size | | | |
| | ≤ 2 cm | 9 (31.0) | 0 (0.0) |
| | > 2 cm | 20 (69.0) | 11 (100.0) |
| Muscle layer invasion | | | |
| | < 50% | 24 (82.8) | 0 (0.0) |
| | ≥ 50% | 5 (17.2) | 11 (100.0) |
| Cervical invasion | | | |
| | Present | 0 (0.0) | 4 (36.4) |
| | Absent | 29 (100.0) | 7 (63.6) |
| Lymph node metastasis | | | |
| | Present | 8 (27.6) | 7 (63.6) |
| | Absent | 21 (72.4) | 4 (36.4) |

| | | | |
|---|---|---|---|
| *Including mixed type of endometrioid carcinoma and non-endometrioid carcinoma | | | |

### (2) RNA extraction and purification

In order to perform CAGE analysis on the specimens of 40 cases obtained in (1), totalRNA was extracted. totalRNA was extracted using Trizol (registered trademark) reagent (manufactured by Thermo Fisher Scientific) and RNeasy (registered trademark) Plus Mini Kit (manufactured by QIAGEN) according to the attached protocol. Ultraviolet absorption (230, 260, 280 nm) of the extracted RNA was measured with a spectrophotometer (NanoDrop (trademark), manufactured by Thermo Fisher Scientific), and the concentration and 260/280 ratio were calculated to confirm that the 260/280 ratio was 1.9 to 2.2.

### (3) Preparation of CAGE library

Using purified RNA, a CAGE library was prepared by non-amplified untagged CAGE method ("Cell Engineering Supplementary Book, Next Generation Sequencer, Advanced Methods by Purpose", supervised by Sumio Kanno, Yutaka Suzuki, published by Gakken Medical Shujunsha Co., Ltd., September 19, 2012), see Chapter 3, 3, "Comprehensive promoter analysis (non-amplified CAGE method using Illumina sequencer)"). Specifically, the purified RNA was subjected to a reverse transcription reaction by SuperScript (registered trademark) III reverse transcriptase (manufactured by Thermo Fisher Scientific)for purification, then diol of ribose was oxidized with sodium periodatefor aldehydation, and biotin was added to the aldehyde group by adding biotin hydrazide (manufactured by Vector Laboratories). After the single-stranded RNA portion was digested and purified by RNase I (manufactured by Promega Corporation), only the RNA/cDNA double strand biotinylated by avidin magnetic beads (Dynabeads (registered trademark) M-270 streptavidin manufactured by Thermo Fisher Scientific) was bound to the bead surface, and cDNA was liberated and recovered by RNase I/H digestion and heat treatment, and purified by AMPure XP (manufactured by Beckman Coulter, Inc.). Double-stranded cDNA library was prepared using Deep Vent (exo-) DNA Polymerase (manufactured by NEW ENGLAND BioLabs) after connecting the necessary adaptors necessary for sequencing to both ends of the recovered cDNA. The prepared CAGE library was sequenced by HiSeq2500 manufactured by Illumina. Note that the standard condition of AMPure XP used for purification, buffer substitution, and the like in this step is a condition under which a nucleic acid having a length of 100 bases or more is recovered in the case of a double strand, and the CAGE library produced by this step adopting this consists of double-stranded DNA having a strand length of 100 bases or more.

### (4) RNA expression analysis

### i) Preparation of reference transcription start site

Among transcription start sites identified in "FANTOM5" (Nature. 2014 Mar 27; 507(7493): 462-70)), which is a project in which the activity of the transcription start point was measured genome-wide for a total of about 1000 human samples including human primary cultured cells, cell lines, and further tissues and the like, about 180,000 transcription start sites defined on human reference genome hg19 were used as reference transcription start sites.

### ii) Quantification of transcriptional activity

Among the reads obtained by sequencing, reads containing N as a base were removed, an rRNA sequence (GenBank accession No. U13369.1) was further removed by rRNAdust60, and alignment of the remaining reads with a human reference genome (hg19) was performed using Burrows-Wheeler Aligner61. Reads with a mapping quality of less than 20 were removed using SAM tools (Nat Protoc. 2009; 4(1) : 44-57). Only alignments were selected such that the start position of the alignment was located within the reference transcription start site, and the number of reads for each transcription start site was enumerated. The counts were converted to counts per million using the total number of reads of each library and the library size estimated by RLE (Genome Biol. 2010; 11(10): R106) method.

### iii) TCGA data preparation

Clinical records and RNA-seq analysis results of The Cancer Genome Atlas (TCGA) were obtained from Genomic Data Commons (GDC) Data Portal (http://gdc.nci.nih.gov/) of the National Institutes of Health (NIH).

### (5) Results

Difference analysis on the transcriptional activity quantified above between a profile group derived from clinical specimens obtained from the "metastasis-negative group of the low to moderate risk group for recurrence" and a profile group derived from clinical specimens obtained from the "metastasis-positive group of the high risk group and the low to moderate risk group for recurrence" using R/Bioconductor edgeR package (Bioinformatics. 2010 Jan 1; 26(1): 139-40). That is, this is to statistically test whether an average expression level differs between the two groups (equality of the average expression level is defined as null hypothesis, and assuming that this null hypothesis is true, the probability of accidental occurrence is calculated). When the threshold was set to FDR (false discovery rate) of 5%, six DNAs containing a smaller transcription start site were identified. These were six variants of Progesterone receptor (PGR) gene (PGR variant p7: SEQ ID NO: 1, PGR variant p2: SEQ ID NO: 2, PGR variant p4: SEQ ID NO: 3, PGR variant p6: SEQ ID NO: 4, PGR variant p1: SEQ ID NO: 5, PGR variant p11: SEQ ID NO: 6). This criterion is based on the statistical presumption that 95% of candidates extracted by the corresponding threshold value truly have expression difference, and is stricter than the P value (the probability of accidental occurrence when it is assumed that there is no expression difference) of 5% usually used widely.

The expression levels of six variants of these PGR genes were significantly higher in the metastasis-negative group of the low to moderate risk group for recurrence than in the metastasis-positive group of the high risk group and the low to moderate risk group, respectively (Fig. 2).

In addition, even when the six variants were grouped, the expression level was significantly higher in the metastasis-negative group of the low to moderate risk group for recurrence than in the metastasis-positive group of the high risk group and the low to moderate risk group (Fig. 3). As a result of confirming the RNA expression level in the TCGA data, it was similarly confirmed that the expression level of PGR was significantly higher in the metastasis-negative group of the low to moderate risk group for recurrence than in the metastasis-positive group of the high risk group and the low to moderate risk group (Fig. 4).

Furthermore, as a result of examining the TCGA data, it became clear that the RNA expression level of estrogen receptor α (ERα) was also significantly higher in the metastasis-negative group of the low to moderate risk group for recurrence than in the metastasis-positive group of the high risk group and the low to moderate risk group (Fig. 4). On the other hand, the RNA expression levels of other steroid hormone receptors such as estrogen receptor β (ERβ) and androgen receptor (AR) were not significantly different between both groups (Fig. 4).

Differences in expression levels between groups were assessed with Mann-Whitney U test. Statistical analysis and graph creation were performed using R statistical processing software ver 3.2.2 (http://www.R-project.org). p < 0.05 was defined as significant difference.

In general recurrence risk classification, risk factors include the presence or absence of lymph node metastasis, and lymph node metastasis positive is classified into a high risk group as an extrauterine lesion. However, in this study, a modified recurrence risk classification (Fig. 1) in which lymph node metastasis is excluded from risk factors is used in the development of a companion marker for the method for assessing lymph node metastatic potential. Therefore, in this example, as a true comparison between the low to moderate risk group for recurrence and the high risk group for recurrence, "the metastasis-negative group of the low to moderate risk group for recurrence" and "the metastasis-positive group of the high risk group and the low to moderate risk group for recurrence" are compared. Accordingly, PGR and ERa were considered to be useful as markers for stratifying the low to moderate risk group and the high risk group for recurrence of endometrial cancer by their expression levels.

Example 2 Determination of recurrence risk of endometrial cancer using PGR and ERα as markers 1

### (1) Specimen

Paraffin-embedded samples of main lesions of 115 cases of endometrial cancer in Table 1 were used. The breakdown of the recurrence risk for the 115 cases is shown in Table 3.

**[Table 3]**

| | | Recurrence risk | |
|---|---|---|---|
| Factor | | Low to moderate risk (%) | High risk (%) |
| Case | | 85 | 30 |
| Age (average - range) | | 54 (36-79) | 62 (31-85) |
| Menopause | | 48 (56.4) | 21 (70.0) |
| Histological type | | | |
| | Endometrioid carcinoma G1 | 68 (80.0) | 9 (30.0) |
| | Endometrioid carcinoma G2 | 13 (15.3) | 5 (16.7) |
| | Endometrioid carcinoma G3 | 4 (4.7) | 5 (16.7) |
| | Other histological type | 0 (0.0) | 11* (36.6) |
| Tumor size | | | |
| | ≤ 2 cm | 21 (24.7) | 3 (10.0) |
| | > 2 cm | 64 (75.3) | 27 (90.0) |
| Muscle layer invasion | | | |
| | < 50% | 64 (75.3) | 3 (10.0) |
| | ≥ 50% | 21 (24.7) | 27 (90.0) |
| Cervical invasion | | | |
| | Present | 0 (0.0) | 7 (23.3) |
| | Absent | 85 (100.0) | 23 (76.7) |
| Lymph node metastasis | | | |
| | Present | 8 (9.4) | 10 (33.3) |
| | Absent | 77 (90.6) | 20 (66.7) |

| | | | |
|---|---|---|---|
| *Including mixed type of endometrioid carcinoma and non-endometrioid carcinoma | | | |

### (2) Protein detection by immunohistochemical staining

The paraffin-embedded sample of (1) was sliced into tissue pieces with a thickness of 4 um and attached to a slide glass to obtain a section sample. For each specimen, one section sample was stained with hematoxylin/eosin (HE), and the presence of endometrial cancer tissue was confirmed for all specimens. Next, another section sample of each specimen was subjected to immunohistochemical staining according to a standard protocol using an automatic staining system (VENTANA BenchMark ULTRA) manufactured by Ventana. Specifically, an activation solution (manufactured by Ventana) was added to the section, and the resultant was heated at 100°C for 60 minutes with a slide heater to activate antigen. Further, the slides were immersed in 3% H₂O₂ for 4 minutes to inactivate endogenous peroxidase. Then, the resultant was reacted with an anti-PGR antibody (Ventana CONFIRM PR (1E2), product number 790-2223, manufactured by Ventana) or an anti-ERα antibody (Ventana CONFIRM ER (SP1), product number 790-4324, manufactured by Ventana) at 42°C for 32 minutes to perform a primary antibody reaction. Thereafter, using ultraView DAB universal kit (product number 760-500, manufactured by Ventana), a secondary antibody reaction and a staining reaction were performed according to the protocol attached to the kit.

The obtained specimens were observed under a microscope by two pathologists, and the immunostaining result was assessed by Allred score. Differences in expression levels between groups were assessed with Mann-Whitney U test. Statistical analysis, AUC calculation, and graph creation were performed using R statistical processing software ver 3.2.2 (http://www.R-project.org). p < 0.05 was defined as significant difference.

### (3) Results

1) PGR and ERa showed a staining difference between the low to moderate risk group and the high risk group for recurrence, respectively, and the Allred score was significantly higher in the low to moderate risk group than in the high risk group (Fig. 5(A), p < 0.001). Further, as a result of calculating sensitivity and specificity of PGR and ERa to create ROC curves, the AUC was 76.2% for PGR and 77.0% for ERa (Fig. 6(A)). Therefore, it was confirmed that PGR and ERa were capable of discriminating between the low to moderate risk group and the high risk group for recurrence of endometrial cancer as markers.
2) As a result of comparing the group of endometrioid carcinoma G1 with the other groups, PGR and ERα each showed a staining difference between both groups, and the Allred score was significantly higher in the G1 group than in the other groups (Fig. 5(B), p < 0.001). Further, as a result of calculating sensitivity and specificity of PGR and ERα to create ROC curves, the AUC was 78.7% for PGR and 73.4% for ERa (Fig. 6(B)). Therefore, it was confirmed that PGR and ERa can discriminate the tissue malignancy of endometrial cancer (G1 and others) as markers.
3) As a result of comparing the group of endometrioid carcinoma G1 or G2 with the other groups, PGR and ERα each showed a staining difference between both groups, and the Allred score was significantly higher in the G1 or G2 group than in the other groups (Fig. 5(C), p < 0.001). Further, as a result of calculating sensitivity and specificity of PGR and ERa to create ROC curves, the AUC was 85.1% for PGR and 84.8% for ERa (Fig. 6(C)). Therefore, it was confirmed that PGR and ERa can discriminate the tissue malignancy of endometrial cancer (G1/G2 and others) as markers. The AUC by the sum of the Allred scores of PGR and ERa was 86.6%, which was equivalent to that of PGR and ERa alone. Therefore, it is considered that PGR and ERα have sufficient discrimination potential even when PGR and ERa are used alone.

Since the difference in prognosis is larger between G2 and G3 than between G1 and G2, it is more practically useful to discriminate between G1/G2 and other tissue malignancy than to discriminate between G1 and other tissue malignancy.

Example 3 Determination of recurrence risk of endometrial cancer using PGR and ERα as markers 2

### (1) Specimen

Specimens collected from 115 cases in Table 1 were used.

### (2) Measurement of PGR and ERa expression levels using RT-qPCR

### 1) RNA extraction and purification

The stored specimen was divided into 50 mg or less and placed in a 2 mL sample tube, and zirconia beads ϕ2.0 and an RNA extraction reagent (RNeasy (registered trademark) Mini Kit (manufactured by QIAGEN) were added and homogenized at 13500 rpm for 3 minutes using a bead cell disruptor (Micro Smash MS-100, manufactured by TOMY). Next, RNA extraction was performed according to the protocol of the RNA extraction reagent. Ultraviolet absorption (230, 260, 280 nm) of the extracted RNA was measured with a spectrophotometer (Nano Drop, manufactured by Thermo Fisher Scientific), and 260/230 ratio and 260/280 ratio were confirmed to be 1.9 to 2.2.

### 2) Purification of cDNA

First strand cDNA was synthesized from 2000 ng of extracted RNA derived from the surgically extracted specimen extracted in 1), according to the protocol of PrimeScript^{™} 1st strand cDNA Synthesis Kit (manufactured by TAKARA).

### 3) Measurement of PGR expression level and ERa expression level by RT-qPCR

A reaction solution was prepared according to the protocol of Fast SYBR (registered trademark) Green Master Mix (manufactured by Applied Biosystems), and RT-qPCR was performed using Life technologies 7500 Fast Real-Time PCR System, using the following primers.
a) PgR primer (TAKARA Primer Set ID: HA278655)
b) ERa primer (TAKARA Primer Set ID: HA271010)
c) SUDS3 primer (TAKARA Primer Set ID: HA237699)

For the gene expression level, the Ct value of SUDS3 quantified as a control gene was subtracted from the Ct value of PGR or ERa and the obtained value was multiplied by -1 to calculate a -ΔCt value, and a statistically significant difference between each two groups of the low to moderate risk group and the high risk group, the G1 group and the other histological groups, and the G1 or G2 group and the other histological groups was assessed with unpaired student t-test. The SUDS3 selected as the control gene (housekeeping gene) was confirmed as a gene having a small difference in expression level between the two groups compared. In this quantitative test for both genes, it was confirmed that PCR amplification efficiency was almost constant.

### (3) Results

1) The expression levels of PGR and ERα were each significantly higher in the low to moderate risk group than in the high risk group for recurrence (Fig. 7(A), p = 1.23 × 10⁻⁵, p = 1.84 × 10⁻⁴) . Further, as a result of calculating sensitivity and specificity of PGR and ERα to create ROC curves, the AUC was 77.5% for PGR and 74.8% for ERa (Fig. 8(A)). Therefore, it was confirmed that PGR and ERa were capable of discriminating between the low to moderate risk group and the high risk group for recurrence of endometrial cancer as markers.
2) As a result of comparing the group of endometrioid carcinoma G1 with the other groups, the expression levels of PGR and ERα were each significantly higher in the G1 group than in the other groups (Fig. 7(B), p = 4.52 × 10⁻⁷, p = 1.51 × 10⁻⁷). Further, as a result of calculating sensitivity and specificity of PGR and ERα to create ROC curves, the AUC was 77.7% for PGR and 81.6% for ERa (Fig. 8(B)). Therefore, it was confirmed that PGR and ERa can discriminate the tissue malignancy of endometrial cancer (G1 and others) as markers.
3) As a result of comparing the group of endometrioid carcinoma G1 or G2 with the other groups, the expression levels of PGR and ERα were each significantly higher in the G1 or G2 group than in the other groups (Fig. 7(C), p = 1.47 × 10⁻⁷, p = 2.54 × 10⁻⁷). Further, as a result of calculating sensitivity and specificity of PGR and ERα to create ROC curves, the AUC was 82.9% for PGR and 88.2% for ERa (Fig. 8(C)). Therefore, it was confirmed that PGR and ERa can discriminate the tissue malignancy of endometrial cancer (G1/G2 and others) as markers.

Example 4 Assessment of lymph node metastasis of endometrial cancer by combining marker for risk assessment for recurrence of endometrial cancer and marker for assessment of lymph node metastatic potential of endometrial cancer

### (1) Specimen

Specimens collected from 115 cases in Table 1 were used.

### (2) Measurement of SEMA3D and TACC2 expression levels using RT-qPCR

In the same manner as in Example 3 (2), RT-qPCR was performed for SEMA3D and TACC2, and the Ct value of SUDS3 quantified as a control gene was subtracted from the Ct value of SEMA3D or TACC2 isoform L or S and the obtained value was multiplied by -1 to calculate a -ΔCt value. The following primers were used.
d) SEMA3D primer (TAKARA Primer Set ID: HA239516)
e) TACC2 isoform L or S primer:
   Primer F: CCAgTTgCTgAAgggCAgAA (SEQ ID NO: 18)
   Primer R: gCggACCTTggAgTCTgAg (SEQ ID NO: 19)

### (3) Analysis

Using the data (-ΔCt value) of the expression levels of SEMA3D and TACC2 obtained in (2), and using all of these data and the data (-ΔCt value) of the expression levels of PGR and ERa obtained in Example 3, the prediction probability was calculated by logistic regression model with respect to the separation potential between ymph node metastasis positive and negative. Statistical analysis and graph creation were performed using R statistical processing software ver 3.2.2.

### (4) Results

The results are shown in Fig. 9. The AUC was 79.442% for the two markers SEMA3D and TACC2 (Fig. 9A), whereas the addition of the two markers PGR and ERa to SEMA3D and TACC2 improved the AUC to 83.869% (Fig. 9B). It was confirmed that the separation potential between lymph node metastasis positive and negative by the two markers of SEMA3D and TACC2 was improved by further using the two companion markers of PGR and ERα.

### Industrial Applicability

According to the present invention, it is possible to objectively determine recurrence risk of endometrial cancer by examining the primary lesion of the endometrium collected before or during surgery. This makes it possible to select endometrial cancer with low to moderate risk of recurrence. Furthermore, by using the selected endometrial cancer as a subject, it is possible to determine the presence or absence of lymph node metastasis and predict the occurrence of endometrial cancer with higher accuracy. As a result, unnecessary lymph node dissection can be avoided, and complications such as postoperative lymphedema can be reduced. That is, the marker of the present invention enables selection of a subject suitable for assessment of lymph node metastasis of endometrial cancer, and is useful as a companion marker contributing to improvement in accuracy of the assessment.

## Claims

1. A method for assessing recurrence risk of endometrial cancer, comprising a step of measuring an expression level of at least one selected from the group consisting of progesterone receptor (PGR) and estrogen receptor α (ERα) in a biological sample derived from a cancer tissue isolated from an endometrial cancer patient.

2. The method according to claim 1, comprising the following steps a and b:
a) a step of measuring the expression level of at least one selected from the group consisting of PGR and ERa in the biological sample derived from the cancer tissue isolated from the endometrial cancer patient; and
b) a step of comparing the expression level of at least one selected from the group consisting of PGR and ERa with a control level or a threshold level.

3. The method according to claim 1 or 2, wherein when the expression level of at least one selected from the group consisting of PGR and ERa is increased above the control level or the threshold level, it is determined that the recurrence risk is low risk or moderate risk.

4. The method according to any one of claims 1 to 3, wherein the expression level of at least one selected from the group consisting of PGR and ERa in the biological sample is an amount of transcription product or translation product of at least one selected from the group consisting of PGR and ERa in the endometrial cancer tissue.

5. A method for assessing tissue malignancy of endometrial cancer, comprising a step of measuring an expression level of at least one selected from the group consisting of PGR and ERa in a biological sample derived from a cancer tissue isolated from an endometrial cancer patient.

6. A method for assessing lymph node metastasis or lymph node metastatic potential or prognosis of endometrial cancer, comprising a step of measuring, in a biological sample derived from a cancer tissue isolated from an endometrial cancer patient, an expression level of at least one selected from the group consisting of PGR and ERa and an expression level of at least one selected from the group consisting of SEMA3D and TACC2.

7. The method according to claim 6, comprising the following steps c to e:
c) a step of measuring the expression level of at least one selected from the group consisting of PGR and ERa and the expression level of at least one selected from the group consisting of SEMA3D and TACC2 in the biological sample derived from the cancer tissue isolated from the endometrial cancer patient;
d) a step of comparing the expression level of at least one selected from the group consisting of PGR and ERa with a control level or a threshold level; and
e) a step of comparing the expression level of at least one selected from the group consisting of SEMA3D and TACC2 with a control level or a threshold level.

8. The method according to claim 6 or 7, wherein when the expression level of at least one selected from the group consisting of PGR and ERa is increased above the control level or the threshold level, and the expression level of SEMA3D is increased above the control level or the threshold level, the expression level of TACC2 is decreased below the control level or the threshold level, or the relative expression level when comparing the expression level of SEMA3D with the expression level of TACC2 is increased above the control level or the threshold level, it is determined that there is no lymph node metastasis or the lymph node metastatic potential is low or prognosis is good.

9. The method according to claim 6, comprising the following steps f to j:
f) a step of measuring the expression level of at least one selected from the group consisting of PGR and ERa in the biological sample derived from the cancer tissue isolated from the endometrial cancer patient;
g) a step of comparing the expression level of at least one selected from the group consisting of PGR and ERa with a control level or a threshold level;
h) selecting a biological sample in which the expression level of at least one selected from the group consisting of PGR and ERa is increased above the control level or the threshold level;
i) measuring the expression level of at least one selected from the group consisting of SEMA3D and TACC2 in the biological sample selected in h); and
j) comparing the expression level of at least one selected from the group consisting of SEMA3D and TACC2 with a control level or a threshold level.

10. The method according to claim 9, wherein when the expression level of SEMA3D is increased above the control level or the threshold level, the expression level of TACC2 is decreased below the control level or the threshold level, or the relative expression level when comparing the expression level of SEMA3D with the expression level of TACC2 is increased above the control level or the threshold level, it is determined that there is no lymph node metastasis or the lymph node metastatic potential is low or prognosis is good.

11. The method according to any one of claims 6 to 10, wherein the expression level of at least one selected from the group consisting of PGR and ERa in the biological sample is an amount of transcription product or translation product of at least one selected from the group consisting of PGR and ERa in an endometrial cancer tissue, and the expression levels of SEMA3D and TACC2 in the biological sample are amounts of the transcription products or translation products of SEMA3D and TACC2 in the endometrial cancer tissue.

12. A test kit for assessing recurrence risk of endometrial cancer or tissue malignancy of endometrial cancer used in the method according to any one of claims 1 to 5, comprising at least one antibody selected from the group consisting of an antibody that specifically binds to a PGR protein and an antibody that specifically binds to an ERa protein, or at least one oligonucleotide selected from the group consisting of an oligonucleotide that specifically recognizes a nucleic acid derived from a PGR gene and an oligonucleotide that specifically recognizes a nucleic acid derived from an ERa gene.

13. A test kit for assessing lymph node metastasis or lymph node metastatic potential or prognosis of endometrial cancer used in the method according to any one of claims 6 to 11, comprising at least one antibody selected from the group consisting of an antibody that specifically binds to a PGR protein and an antibody that specifically binds to an ERa protein, and at least one antibody selected from the group consisting of an antibody that specifically binds to a SEMA3D protein and an antibody that specifically binds to a TACC2 protein, or at least one oligonucleotide selected from the group consisting of an oligonucleotide that specifically recognizes a nucleic acid derived from a PGR gene and an oligonucleotide that specifically recognizes a nucleic acid derived from an ERa gene, and at least one oligonucleotide selected from the group consisting of an oligonucleotide that specifically recognizes a nucleic acid derived from a SEMA3D gene and an oligonucleotide that specifically recognizes a nucleic acid derived from a TACC2 gene.

14. A device for assessing recurrence risk of endometrial cancer or tissue malignancy of endometrial cancer, used in the method according to any one of claims 1 to 5.

15. A device for assessing lymph node metastasis or lymph node metastatic potential or prognosis of endometrial cancer, used in the method according to any one of claims 6 to 11.

16. Use of at least one selected from the group consisting of PGR and ERα as a marker for assessing recurrence risk of endometrial cancer.
